# EUROPEAN PATENT SPECIFICATION

(11) **EP 3 881 891 B1**
(45) Date of publication and mention of the grant of the patent: **14.06.2023**
(21) Application number: 20164082.8
(22) Date of filing: 19.03.2020
(51) Int. Cl.: A61N 1/36, A61N 1/375

(54) **CONTROL CIRCUIT FOR IMPLANTABLE PULSE GENERATOR**
STEUERUNGSSCHALTUNG FÜR EINEN IMPLANTIERBAREN IMPULSGENERATOR
CIRCUIT DE COMMANDE POUR GÉNÉRATEUR D'IMPULSIONS IMPLANTABLE

(43) Date of publication of application: 22.09.2021
(73) Proprietor: ONWARD Medical N.V., 5611 ZX Eindhoven (NL)
(72) Inventor: WEIJAND, Koen, Eindhoven, 5656AE (NL); TOL, Jeroen, Eindhoven, 5656AE (NL)
(74) Representative: DTS Patent- und Rechtsanwälte Schnekenbühl und Partner mbB

(56) References cited:
- WO-A1-99/08749
- US-A- 5 876 425
- US-A1- 2011 093 043
- US-A1- 2019 299 006
- US-B2- 9 717 908

## Description

The present invention relates to a control circuit for an implantable pulse generator.

Control circuits for neurostimulators are known from the prior art.

For example, US 5,876,425 discloses a power control loop for implantable tissue stimulators. The power control loop is established between an external control device and an implantable device so that only the amount of power needed by the implant device to sustain its present operating conditions is transmitted across a transcutaneous transmission link, thereby reducing the amount of power expended by the external control device.

US 6,901,292 discloses a control of externally induced current in an implantable pulse generator. Disclosed is a current limiting apparatus and method for limiting current flow, induced when the level of an external signal is greater than an external signal threshold signal level, in a conductive loop formed by a medical device implanted within a living organism having electrically excitable tissue. The system includes an implantable pulse generator (IPG) system having a housing, a signal generator disposed in the housing that generates an electrical signal, and at least one lead extending from the housing to convey electrical signal to the patient. To limit the induced current flow, the IPG includes current limiting componentry, an impedance increasing element, and/or alternating current blocking elements. These components provide an alternating current impedance path to the electrical ground from a lead coupled to the capacitive element. Also disclosed are techniques for reducing the effective surface area of the current inducing loop caused by the IPG system.

US 7,801,600 discloses controlling of charge flow in the electrical stimulation of tissue. The method includes receiving a charge setting describing an amount of charge that is to flow during a stimulation pulse that electrically stimulates a tissue, and generating and delivering the stimulation pulse in a manner such that an amount of charge delivered to the tissue during the stimulation pulse accords with the charge setting.

US2019/299006A1 discloses a passive tissue biasing circuitry for use with an Implantable Pulse Generator (IPG), said passive tissue biasing circuitry being configured to establish and hold a common mode voltage in a tissue to facilitate sensing of neural responses. In particular, once the common mode voltage is established, voltages accompanying the production of stimulation pulses will be referenced to the common mode voltage to ease neural response sensing. A capacitor is provided between a case and a reference voltage source. The reference voltage source may comprise a constant (or pseudo-constant) voltage, provided by a voltage source.

US2011/093043A1 discloses a device for delivery of electrical stimulation via an electrode on a housing of an implantable medical device (IMD) while substantially simultaneously delivering electrical stimulation via one or more electrodes (having the same polarity as the electrode on the housing) on one or more leads engaged to the IMD. The device may be provided with a stimulation generator including a stimulation control module, a reference voltage source, a switch array, and a current regulator array. The reference voltage source may provide operating power to the current regulator array and may include a regulated voltage that sets the level of the reference voltage. A regulated power source may produce one or more regulated voltage levels for use as reference.

Neurostimulation's inherent zero net charge injection creates the option to set the potential of a tissue node of a stimulation engine in a way that is more power efficient and real-estate friendly than what would be possible if a dedicated supply rail would be implemented to control the stimulation node's potential, for example, through a boost converter or an LDO type of circuit. The tissue node of a stimulation engine can be any node and/or surface configured for delivering and/or controlling stimulation to tissue. Typical examples of such stimulation nodes or surfaces can be the housing or housing portion of a neurostimulator, an electrode of a lead, or any other stimulation node or stimulation surface configured for delivering and/or controlling stimulation to tissue.

This object is achieved with a control circuit for an implantable pulse generator with the features of claim 1. Accordingly, a control circuit for a neurostimulator, especially for an implantable pulse generator for a neurostimulator, comprising and/or being connected to at least one energy source, at least one housing or housing portion and/or at least one stimulation node and/or at least one stimulation surface configured to be in contact with tissue of a patient, at least one capacitor being directly or indirectly connected with the housing or housing portion and/or the stimulation node and/or the stimulation surface, the control circuit and/or the capacitor being arranged and configured such that the housing or housing portion and/or the stimulation node and/or the stimulation surface is kept at a predefined voltage or kept within a predefined voltage range by means of the control circuit and/or the capacitor. The invention is defined by the independent claim 1 and is based on the basic idea that by means of a simple electronic element i.e. a capacitor (or an element with similar function), the housing or at least a housing portion of an IPG (or any other implantable medical unit (IMU)) can be kept at a desired predefined voltage. The housing may be for example the can (also CAN) of the implantable pulse generator. By this the can control can be done for keeping the tissue which is surrounding and/or in galvanic contact with the can of the implantable pulse generator at a desired potential.

In other words, a power-efficient and area and volume claim friendly control of the CAN potential of a neurostimulator is provided. This may immediately set the tissue potential if the CAN is in direct (galvanic) contact with tissue, for example in case of an implantable pulse generator (IPG) for a neurostimulation/neuromodulation system. It may have several advantages over using a dedicated supply for maintaining the potential of a CAN or CAN portion of a neurostimulator:
The control circuit may enable a single high voltage supply rail implementation of the stimulation engine (also STIM engine). This leads to a much-reduced footprint in comparison to a dual-rail supply implementation with both a positive and negative supply rail and CAN connected to ground (GND) (e.g. negative polarity of the battery). This is especially important for an IPG realization with discrete and off-the-shelf integrated circuits (ICs)where a dual-rail solution may have a prohibitively large area and volume claim.

Inter alia, controlling the CAN potential through a dedicated DC-DC converter (e.g. a boost converter or LDO type of circuit) may provide a power penalty, and consequently, a negative impact on battery longevity. For example, for stimulation with perfectly balanced stimulation pulses, ideally, there is no net current flow through the housing (averaged over a stimulation period) and the housing voltage is unchanged after each stimulation pulse. Consequently, there is no need for a DC-DC converter to keep the housing at a desired voltage which saves the otherwise consumed quiescent power consumption of such a converter. More realistically, even if the stimulation pulses have some charge imbalance, the resulting (average) current delivered by a dedicated DC-DC converter will be small in practice, and therefore, the resulting power efficiency of this converter will be poor.

Moreover, typically, a DC-DC converter can only source current which means that if the housing sinks more charge than it sources over a stimulation period, which cannot be excluded beforehand, then in practice, the reservoir output capacitor of the DC-DC converter needs to be regulated or controlled to ensure that the output voltage of the converter does not run away. As shown in this disclosure, this CAN controller can be implemented in such a way that the housing can both sink and source current without the need for a dedicated DC-DC converter at all.

This solution has almost no power penalty as the (added) power consumption of the CAN controller is low as it only comes from turning on and off several switches. A well-defined tissue potential through the CAN controller means that the current sources of the STIM engine output stages will also behave well, that is, they will remain in their high-ohmic compliance region during stimulation leading to well-defined stimulation current pulses.

A single-rail STIM engine without controlling the CAN/tissue potential may consume twice the power to generate biphasic stimulation pulses than a pure dual-rail implementation. This doubling of the power consumption may be an issue with the limited available battery capacity that can be realized in an implant. However, a single-rail design with the CAN controller as possible in a preferred embodiment is almost as power efficient as a dual-rail design but without the (prohibitively) large board area and implant volume claim.

There are also disadvantages to the regulated CAN solution:
The CAN controller is realized through a capacitor as short-duration emulator of an actual supply rail or voltage source. As such, the capacitor voltage needs to be controlled so that it has the desired voltage all the time. Although the control as such is not difficult, because of its simplicity and the fact that the tolerance of the capacitor voltage does not need to be very tight in practice, it still adds extra complexity to the overall STIM engine, while it also puts more constraints on the firmware of the implant.

However, the many and also needed advantages of the regulated CAN proposal outweigh these drawbacks.

The main idea behind this disclosure is that stimulation is charge-neutral in nature and as such the net current flow through each branch (e.g. a wire) of a stimulated network (e.g. electrodes and CAN in tissue) is zero (no DC). Or put differently, the sum of the currents over time through a node of interest (e.g. electrode or CAN) is zero, containing no DC component, as otherwise, for example, electrode corrosion and/or tissue damage would occur for those nodes in contact with tissue. Therefore, in applications where the amount of charge flow is also limited, as is the case for neurostimulation pulses, where a stimulation pulse contains a finite amount of charge, a node of interest can be kept at a desired voltage using a capacitor if the capacitor's voltage is monitored and controlled in such a way that this capacitor remains at the desired voltage.

The housing is in an example embodiment the housing (portion) of a neurostimulator or a component of a neurostimulator. In particular, it can be the housing (portion) or CAN of an implantable pulse generator (IPG).

The most natural point to apply this idea is the housing or CAN of an IPG because by doing so the CAN acts as a reference potential for the tissue, assuming the CAN is in direct contact with the tissue as is normally the case. However, any stimulation node or stimulation surface configured for delivering and/or controlling stimulation, and characterized by substantially net zero charge injection, can be used to apply this idea. Therefore, the wording 'housing', 'housing portion', 'can' or 'CAN' may freely be substituted for 'stimulation node' or 'stimulation surface' and likewise, the wording 'CAN controller', 'can controller' or simply 'controller' can be replaced with 'stimulation node controller' or 'stimulation surface controller'.

The energy source can be a current source and/or a voltage source and/or power source. The energy source can be a battery or DC voltage source. Furthermore, the energy source can be connected directly and/or indirectly with the control circuit. Also, the energy source can be a part of the control circuit or can be separated from it.

The CAN portion may be configured to be in contact with tissue, especially being implanted into the human body. The capacitor may be connected with the CAN portion (directly and/or indirectly). Furthermore, the CAN portion is kept at a desired predefined voltage or (electric) potential by means of the capacitor, such that the housing or housing portion and thus also the surrounding tissue being in contact with the CAN and/or CAN portion is kept at a desired predefined voltage or (electric) potential by means of the capacitor.

Further, the control circuit can comprise at least one energy controlling element, which is configured such that it uses energy from or returns energy to the energy source to keep the housing and/or the housing portion and/or the stimulation node and/or the stimulation surface at a predefined voltage or kept within a predefined voltage range.

In other words, in an example embodiment, the control circuit can comprise at least one energy controlling element, which is configured such that it controls the energy taken from the energy source to keep the housing and/or housing portion and/or the stimulation node and/or the stimulation surface at a predefined voltage or keep it within a predefined voltage range. By this, it is assured that by using the energy source itself, the voltage or (electric) potential of the housing or housing portion is controlled and/or adjusted such that it stays at a predefined voltage or is kept within a predefined voltage range.

Also, it is possible the energy controlling element is configured such that where charging up energy is taken from the energy source but discharging delivers energy back to this source.

In other words, energy from the energy source is used and funneled by the controlling element to control and/or adjust the voltage of the housing (portion) and/or the stimulation node and/or the stimulation surface and/or to supply the energy the housing and/or housing portion and/or the stimulation node and/or the stimulation surface delivers, for example, to tissue such that the housing and/or housing portion and/or the stimulation node and/or the stimulation surface is kept at a predefined voltage or kept within a predefined voltage range.

The energy controlling element can be embodied or its function can be provided by any electronic component being capable to do so. For example, the energy controlling element can be embodied by a (micro)controller or its functionality may be provided by an (already present) (micro)controller.

Furthermore, it is possible that the control circuit further comprises a monitoring element.

In particular, the monitoring element is configured such that by means of the monitoring element the voltage across the capacitor and/or the voltage of the housing and/or housing portion and/or the voltage of the stimulation node and/or the stimulation surface is monitored and/or the current through the capacitor and/or the housing and/or the housing portion and/or the stimulation node and/or the stimulation surface is monitored and/or the voltage and/or the current and/or the power of the energy source is monitored.

The monitoring element is an advantageous additional element, which assists the energy controlling element in its function. As such, the monitoring element monitors the voltage across the capacitor and/or the voltage or (electric) potential of the housing (portion) in order to control or correct the voltage across the capacitor or housing (portion).

By this, a monitoring can be done by the controlling element, especially in real-time. In particular, by this it is possible to monitor the at least one energy source and/or the at least one capacitor and/or the at least one housing (portion). The monitoring can include, but is not limited to, a monitoring of a current and/or a voltage and/or a charge. A more accurate monitoring of the control circuit is possible in this way. The monitoring element can make use of e.g. thresholds where staying within the thresholds is tolerated and no action is initiated and exceeding one or more thresholds will lead to an automatic, semi-automatic or manual correction.

Additionally, it is possible that the voltage across the at least one capacitor is controlled and/or corrected by the energy controlling element with input from the monitoring element. The level of the at least one energy source and/or the voltage across the at least one capacitor and/or the voltage or (electric) potential of the at least one housing (portion) can be thus monitored and/or controlled and/or corrected by means of the energy controlling element with input from the monitoring element.

For example, the monitoring element can be embodied as a sensor, while the control circuit may comprise current sources configured in an H-bridge constellation as an actuator to control the level of the energy source and/or the voltage across the capacitor and/or the voltage of the housing (portion).

Especially, when the control circuit needs to deal with charge imbalanced pulses, monitoring is recommended. The reason for this is the fact that monophasic but also imperfect biphasic pulses are not charge neutral, and therefore can induce some drifting away in time, for example, of the voltage across the at least one capacitor. By doing the monitoring, such drifting away can easily be detected and corrected, when necessary or desired.

Furthermore, the voltage provided by the energy source may be a DC voltage. Such voltage can be easily provided by usual batteries with the batteries acting as an example energy source.

Furthermore, it is possible that the control circuit for the implantable pulse generator is configured and arranged for handling bi-phasic pulses. By this, a broader range of neurostimulation can be provided and is possible.

Additionally, the control circuit may comprise at least one of a current source, a switch, a voltage supply, a ground, and a bridge circuit.

The housing and/or housing portion and/or the stimulation node and/or the stimulation surface can be part of the bridge circuit. Furthermore, the bridge circuit may comprise or be an H-bridge circuit.

Additionally, it is possible that a supply voltage may be within a range from 0 V to approx. 50 V, especially approx. 5 V to approx. 30 V, preferably from approx. 20 V to approx. 30V, and may be supplied either directly or indirectly by the energy source, for example, a voltage supply rail.

Further, the control circuit can comprise at least a capacitor, a monitoring element, a switch being arranged in a branch connecting the capacitor with a voltage supply and/or another voltage source, and a further switch being arranged in a branch connecting the capacitor with another rail.

Moreover, the control circuit can comprise at least one further current source.

Moreover, the control circuit can further comprise a first control switch, and a second control switch connected in series, wherein the first control switch and the second control switch are arranged between the first current source and the second current source.

Also, it is possible that the control circuit may comprise a first current source , a second current source, a first control switch, and a second control switch connected in series, wherein the first control switch and the second control switch are arranged between the first current source and the second current source.

The control circuit further may be arranged and provided such that it comprises a further switch for connecting the capacitor and/or the housing or housing portion to ground and/or another rail.

Furthermore, it is possible that the control circuit is connected either directly or indirectly (e.g. via tissue) to a stimulation generation module of the or an implantable pulse generator, wherein the stimulation generation module is powered from a single energy source.

Also it is possible that if the third control switch is in its open state after the capacitor has been charged or discharged to the defined/desired capacitor voltage by the first current and/or second current source, at least one monophasic or biphasic stimulation pulse is generated by the stimulation generation module such that the capacitor is further charged or discharged leading to a drifting away from the defined/desired capacitor voltage after the pulse has been output.

In a further embodiment it is possible that the bridge circuit may comprise a monitoring element for monitoring the capacitor voltage with a first resistor and a second resistor, the monitoring element is connected to the first branch between the first current source and the second current source and is further connected to the ground.

The monitoring element may also monitor the level of the energy source, for example, the control circuit may comprise a voltage source whose voltage level may be monitored by the monitoring element.

Additionally, it is possible that if the first and second control switch are in their open state and the third control switch is in its closed state a difference between the actual capacitor voltage and the defined/desired capacitor voltage is monitored by the monitoring branch of the first resistor and the second resistor such that based on the difference the first control switch or the second control switch is closed.

Furthermore, it is possible that the control circuit may be connected either directly or indirectly, for example via tissue, to a stimulation generation module of the implantable pulse generator, wherein the stimulation generation module uses a single voltage source as energy source.

Furthermore, the voltage of the housing (portion) is monitored and controlled/set with the goal to keep at least one stimulation current source of the stimulation generation module within its compliance range during stimulation.

Also, due to the (electric) potential and/or voltage being controlled and/or set at the housing or housing portion and/or the stimulation node and/or the stimulation surface configured to be in contact with tissue of a patient, at least one stimulation current source or output stage of the stimulation generation module remains in its high-ohmic output range and/or its compliance region during stimulation. If the output stage is voltage driven, instead of current driven via a current source, the control circuit can maintain the desired/defined voltage between the inherent voltage source of the at least one voltage driven output stage and the housing (portion) in contact with tissue Furthermore, in conjunction with the above-described circuit, the following method of controlling a neuromodulation and/or neurostimulation system, especially an implantable pulse generator for a neurostimulation and/or neurostimulation system is disclosed without forming part of the invention.

Accordingly, a method for controlling an implantable pulse generator is provided, comprising the following steps:
- providing at least one energy source,
- providing at least one housing or housing portion configured to be in contact with tissue of a patient,
- providing at least one capacitor being directly or indirectly connected with the housing or housing portion,
- keeping the housing or housing portion at a desired predefined voltage or within a predefined voltage range by means of the capacitor.

The method may be further arranged and conducted such that it comprises the following steps:
- providing the control circuit for the implantable pulse generator comprising the bridge circuit including the capacitor, wherein the capacitor and/or the bridge circuit being connected to the outer housing (portion) of the implantable pulse generator staying in direct contact with tissue; and
- monitoring and/or controlling the voltage of the capacitor by the bridge circuit such that the housing voltage of the outer housing (portion) is directly or indirectly set by the capacitor voltage.

It is possible that the housing voltage is kept at at least one defined housing voltage (or within a predefined voltage range) if the capacitor voltage is also kept at at least one defined capacitor voltage (or predefined voltage range), wherein the housing voltage and the capacitor voltage are different from or substantially equally to each other.

Additionally, it is possible that after the capacitor has been charged or discharged to the defined/desired capacitor voltage that at least one stimulation pulse is generated such that the capacitor is further charged or discharged after the stimulation pulse has been output. This arising difference between the actual capacitor voltage and the defined/desired capacitor voltage may be monitored such that in response to that difference the capacitor is charged or discharged to bring the capacitor voltage back to or near the defined/desired capacitor voltage.

The invention is now described in connection with the drawings.

It is shown in
- Fig. 1A: a generalized node voltage controller in a form of a regulated CAN controller, forming an embodiment of a control circuit according to the present invention, with which the method according to the present invention may be performed;
- Fig. 1B: a second embodiment of a control circuit according to the present invention, with which the method according to the present invention may be performed;
- Fig. 2A: a further schematic illustration of a control circuit according to the present invention according to Fig. 1B, with which the method according to the present invention may be performed;
- Fig. 2B: a third embodiment of a control circuit according to the present invention, with which the method according to the present invention may be performed;
- Fig. 3: a fourth embodiment of a control circuit according to the present invention, with which the method according to the present invention may be performed;
- Fig. 4: a further embodiment of a control circuit according to the present invention, with which the method according to the present invention may be performed; and
- Fig. 5: a further embodiment of a control circuit according to the present invention, expanded with a second monitoring element, with which the method according to the present invention may be performed.

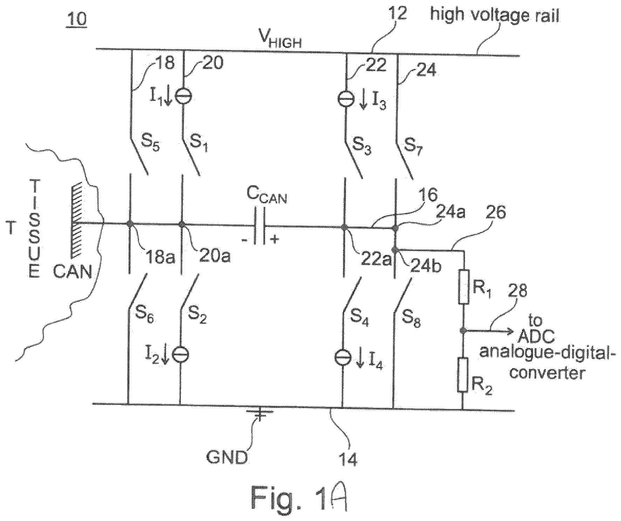

**Fig. 1A** shows a generalized node voltage controller in a form of a regulated CAN controller, forming an embodiment of a control circuit 10 according to the present invention, with which the method according to the present invention may be performed.

The control circuit 10 comprises a high voltage rail 12.

Furthermore, there is another rail 14 on the other side, which is connected to ground (GND).

Additionally, there is a so-called H-bridge 16 in the middle.

Furthermore, the high voltage rail 12 is connected with the other rail 14 by means of four branches, 18, 20, 22 and 24.

Each branch 18, 20, 22, 24 has a node with the H-bridge main branch 16, denoted as node 18a, 20a, 22a and 24a.

In branch 18, there is a switch S5 in the branch segment coming from the high voltage rail 12 and before node 18a.

After node 18a, there is a further switch S₆ in the direction of branch 18, arranged between node 18a and the other rail 14.

In branch 20 there is a first current source I₁, being arranged between the high voltage rail 12 and a first switch S₁ in branch 20.

After switch S₁ there is node 20a, followed by a second switch S₂, which is arranged between node 20a and the other rail 14.

Between switch S₂ and rail 14, there is a second current source I₂.

In branch 22 there is another current source I₃, which is arranged between the high voltage rail 12 and a switch S₃.

The switch S₃ is arranged between node 22a and current source I₃.

After node 22a there is a further switch S₄ in the direction of branch 22, which is arranged between the node 22a and a third current source I₄, which is arranged between switch S₄ and rail 14.

In branch 24, there is a switch S₇ between high voltage rail 12 and node 24a.

After node 24a there is another node 24b, which goes to a further branch 26.

Following branch 24, there is a further switch S₈ between node 24b and rail 14.

In branch 26, there are resistors R₁ and R₂ and between resistors R₁ and R₂, there is a branch 28 going to an analog-digital-converter (ADC).

In branch 16 of the H-bridge and between nodes 20a and 22a, there is a capacitor C_{CAN},

Branch 16 is extending over nodes 22a, 20a, 18a vis-à-vis the tissue T and forms a part of the housing CAN, which is in conductive/galvanic contact with the tissue T.

Rail 14 is connected to ground GND.

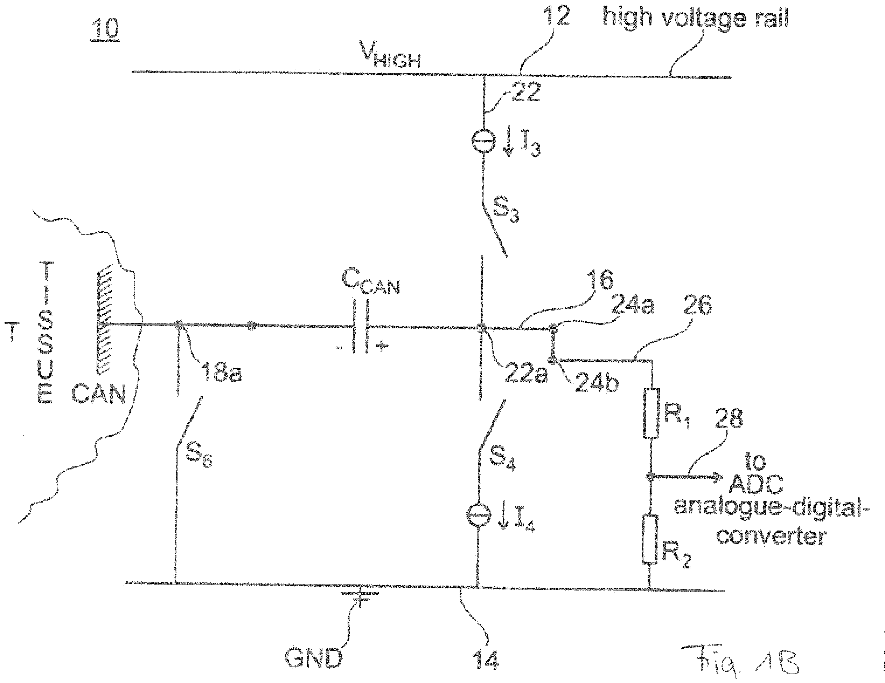

The regulated CAN controller circuit works as follows:
- In its most simple form, only current source I₃ with its enable switch S₃, I₄ with its enable switch S₄ and switch S₆ are present and all other components are removed except for CAN capacitor C_{CAN} and resistive divider R₁ and R₂ as shown in **Fig. 1B**;
- Initially, when the CAN capacitor C_{CAN} is not charged at all, switch S₆ is closed (made conducting) and the capacitor is charged up through enabled current source I₃ (switch S₃ is closed, that is, made conducing) to a desired voltage level. This voltage level can be monitored through the (high-ohmic) resistive divider R₁ and R₂ whose (low-voltage) output goes to a digital-to-analogue converter (ADC), for example, available as part of a microcontroller unit (MCU, not shown, also forming the energy controlling element) of an IPG;
- Next, switch S₆ is opened (made non-conducting) and (enabled) current source I₃ is set to a current level so that it enters its (low-ohmic) linear region and as such, the current source acts as a switch itself, connecting the CAN capacitor C_{CAN} to the high voltage rail V_{HIGH} (provided through a boost converter connected to the IPG battery, the energy source of the IPG). If current source I₃, acting as a switch, has a too high on-resistance, a separate switch S₇ can be connected in parallel to current source I₃ and its switch S₃ and this (very low-ohmic) switch can be closed instead of using current source I₃ (that can be turned off by opening switch S₃ after the CAN capacitor has been charged up to the desired voltage if switch S₇ is added);
- With the CAN at the desired voltage, at least at a level so that all electrode current sources of the output stages of the STIM engine (not shown) are in their high-ohmic output regime, that is, in their compliance region, a stimulation pulse can be generated. For unipolar stimulation through the CAN and a biphasic stimulation current pulse, the CAN capacitor C_{CAN} is charged up further during one phase of the biphasic stimulation current pulse when the CAN sources current to the tissue with this delivered current to the tissue drawn from the high-voltage supply V_{HIGH}. However, the CAN capacitor C_{CAN} is discharged again, not taking any charge from the high-voltage supply V_{HIGH}, when the CAN sinks current from the tissue during the other phase of the biphasic stimulation current pulse, and ideally, in the case of a perfectly balanced biphasic stimulation current pulse with a zero net charge content, the voltage across the capacitor will be the same as before the stimulation pulse was given;
- However, there is always some charge imbalance so there will come a moment that the CAN capacitor voltage has drifted away too much from the intended, desired voltage and the capacitor voltage must be regulated back to this desired voltage. During the grounding phase of the stimulation, when no stimulation pulses are present, current source I₃ is disabled through switch S₃ (making it non-conducting, or S₇ is opened when it was used instead of current source I₃) and switch S₆ is closed again, the CAN capacitor voltage is measured through the resistive divider R₁ and R₂ as before and the MCU switches on either current source I₃ or I₄ depending on whether the CAN capacitor needs to be charged or discharged, resp. to regulate it back to the desired voltage, while monitoring the voltage level through the resistive divider. Ideally, the capacitor has its desired voltage again before the end of the grounding phase but if not, the regulation can continue during the next grounding phase, and the next stimulation pulse or pulses can be given as already explained. In the shown embodiment, the monitoring of the voltage across the CAN capacitor C_{CAN} takes place during the grounding phase of the stimulation period when no pulses are output and is done via branch 26, resistor R1, resistor R2 and the ADC via branch 28. The monitoring, which delivers input to the energy controlling element, makes sure that the housing or housing portion is maintained within and/or adjusted to a desired predefined voltage (range) and/or electrical potential (range) by means of the capacitor, the current sources and the energy source.

There are a lot of variations possible to this theme.

For example, in case of monophasic stimulation, current source I₁, enabled through its switch S₁, can push the same current through the CAN to the tissue as is taken from it through the monophasic stimulation pulse. In this way, ideally, the CAN voltage does not change, and no time needs to be reserved to regulate the capacitor voltage, enabling higher frequency stimulation as it may relax the minimum required grounding time.

In another example, in case of biphasic stimulation, both current source I₁ and current source I₂ can advantageously be used in such a way that the CAN current is not sinked or sourced by the CAN capacitor but by either one of these current sources during the biphasic stimulation pulse, also minimizing the time needed for regulation.

Of course, in the real world, even with the use of current source I₁ and/or I₂, the capacitor voltage must be regulated back to what is desired eventually but this may only be needed after multiple pulses or can be done during multiple grounding phases of short duration enabling higher frequency stimulation.

In yet another example, in case of unipolar stimulation, that is, solely between the stimulation electrodes (not shown) and the CAN, with monophasic pulses, one could close switch S₅ during stimulation and close switch S₆ during grounding while leaving all other switches open as long as this stimulation regime lasts. Any charge accumulated on the CAN capacitor, for example, via leakage current through resistors R₁ and R₂, can be removed during the grounding phase of the stimulation when not only switch S₆ is closed but also switch S₈. In particular, for monophasic, unipolar stimulation, no CAN control is needed at all because the CAN potential can be set without the CAN capacitor.

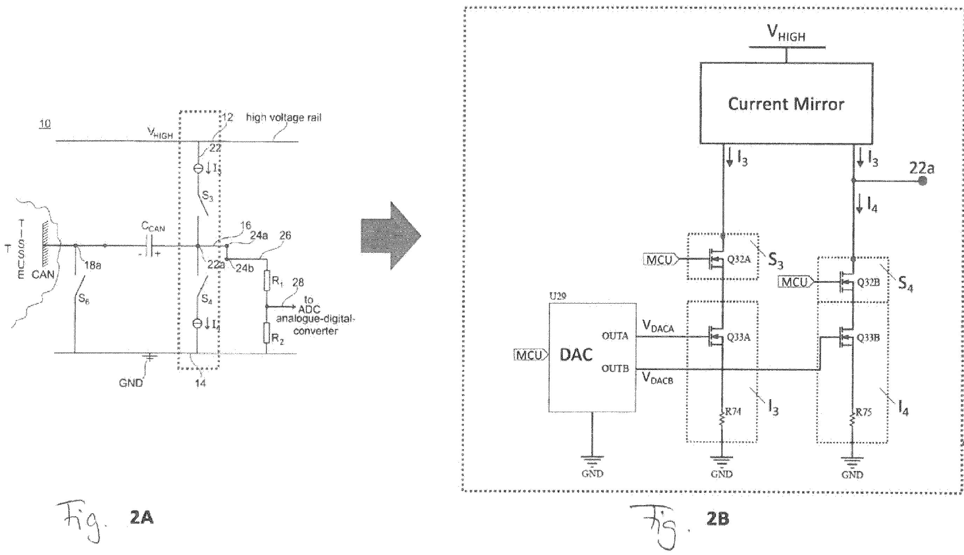

In Fig. 1A, the current sources I₁ and I₂ with their enable switches S₁ and S₂, resp., in the right-hand side branch (branch 22) of the H-bridge, as indicated by the dashed box in **Fig. 2A****,** can be realized with the circuit shown in **Fig. 2B****:**
- Current source I₃ is composed of a series stage with NMOS transistor Q32A and resistor R74. Its output current is determined by output voltage V_{DACA} of output OUTA of (dual) digital-to-analogue converter (DAC) U29. Current source I₄ uses a similar series stage topology of Q33B with R75 connected to output OUTB of the same DAC;
- The DAC output voltages OUT A and OUTB can be set by a MCU, and consequently, via each series stage, the output current of current source I₃ and I₄, resp.;
- Current source I₃ and I₄ can be enabled via NMOS transistor switch Q32A and Q32B, resp. These switches can individually be controlled by the same MCU, The output current of current source I₃ is copied via a current mirror, connected to the high-voltage rail V_{HIGH}, and added to the output current of current source I₄ at node 22a.

All other current sources with their enable switches as shown in Fig. 1A, 1B, 3, 4 and 5 can be realized in the same way. In these figures, the stand-alone switches S₅ and S₇ can each be realized with a single PMOS transistor, while switches S₆ and S₈ can each be realized with a single NMOS transistor.

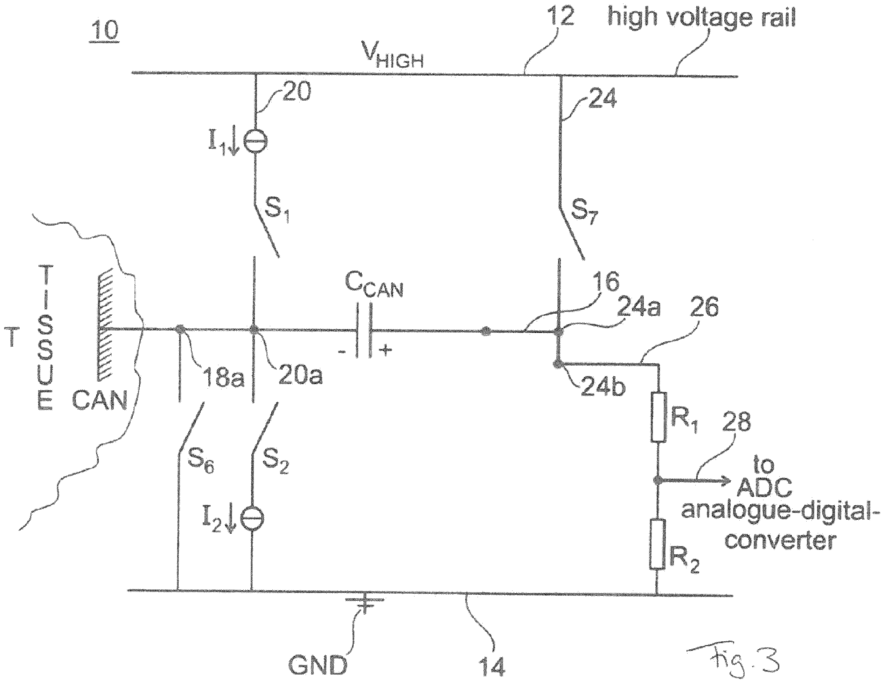

**Fig. 1B** uses the current sources in the right-hand side branch of the H-bridge to regulate the capacitor voltage during the grounding phases of the stimulation but one can also regulate with only the current sources in the left-hand side branch, branch 20, of the H-bridge as shown in **Fig. 3****.**

For example, if the CAN capacitor voltage is too low, current source I₂ can be enabled, via switch S₂, after the grounding phase of the stimulation. This current charges up the capacitor and during the next grounding phase it can be checked if the capacitor voltage is back to the intended level. If not, the regulation process is continued, possibly with a further adjustment of the current of current source I₂. The other way around, if the capacitor voltage is too high, current source I₁ with its switch S₁, can be enabled and controlled to regulate the CAN capacitor voltage back to the intended level.

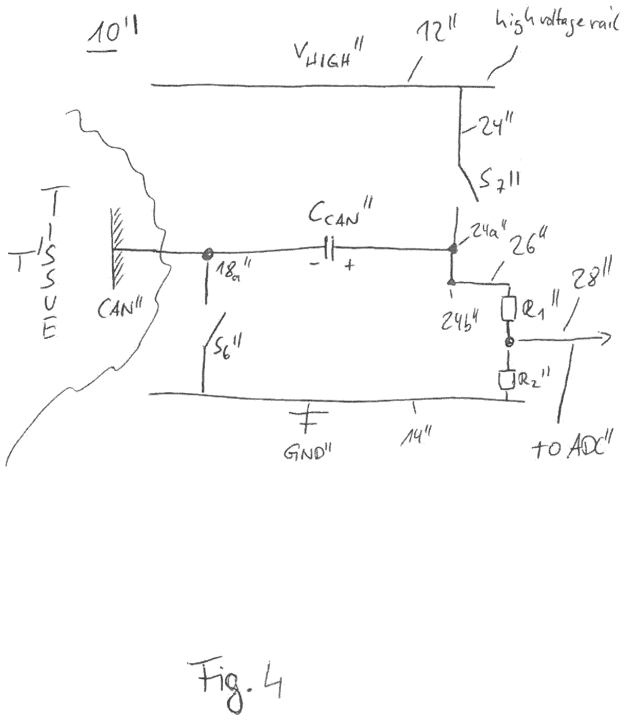

**Fig. 4** shows a further embodiment of a control circuit according to the present invention, with which the method according to the present invention may be performed.

The shown embodiment has the same structural and functional features as control circuit 10 as shown in Fig. 1A. Similar or the same features of the control circuit 10 have the same reference number, however with two added apostrophes (").

The following differences to the embodiment of Fig. 1A exist:
The embodiment of Fig. 4 shows the minimum configuration. The control circuit 10" can already work with only switch S₆", capacitor C_{CAN}", switch S₇", high voltage rail V_{HIGH}" and the monitoring element in branch 26" with its components, R₁", R₂", and the connection to analogue-to-digital converter ADC" via branch 28" if the stimulation pulses themselves are used to regulate the CAN capacitor voltage.

For example, during the grounding phase of the stimulation, when no stimulation pulses are generated and switch S₆" is closed and switch is S₇" is open, the voltage across the CAN capacitor C_{CAN}" is monitored and if it turns out that the capacitor voltage has drifted away from the desired voltage or is outside its desired voltage range, the CAN capacitor voltage is adjusted after the grounding phase via the stimulation pulses themselves. So, the capacitor voltage regulation does not take place during the grounding phases but during the moments that stimulation pulses are present as was also the case in the embodiment of Fig. 3.

For example, if biphasic stimulation pulses are used for the stimulation and the monitored capacitor voltage turns out to be lower than desired, the MCU adjusts the stimulation pulses (e.g. by changing the amplitude and/or duration of a pulse phase) in such a way that the net delivered charge of the pulses to the tissue becomes negative (temporary negative DC current), and consequently, the CAN capacitor is charged and its voltage increased after each stimulation pulse. This process can be monitored during each grounding phase and the regulation process can be stopped (by reverting to the original amplitude and/or duration of the impacted pulse phase) once the CAN capacitor voltage has been charged to its intended voltage level.

The other way around, if the monitored capacitor voltage turns out to be higher than desired, the MCU adjusts the stimulation pulses in such a way that the net delivered charge to the tissue becomes positive (temporary positive DC current) until the CAN capacitor voltage has been discharged to its intended voltage level.

This same mechanism to regulate the CAN capacitor voltage via the stimulation pulses themselves can also be used to fine tune the biphasic pulse parameters (e.g. duration of the pulse phases) in such a way that the pulses become (almost) perfectly charge balanced. This minimizes the CAN capacitor voltage drift, and therefore, the amount of regulation actions.

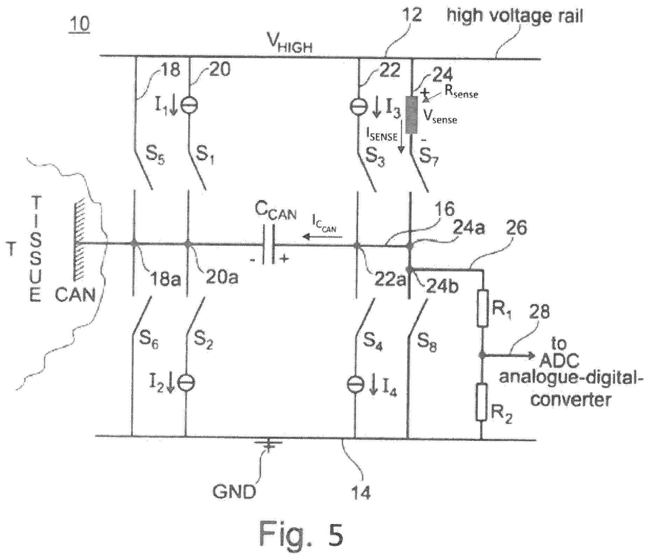

The control circuit of Fig. 1A can be expanded with a second monitoring element that monitors the current flow through the CAN capacitor in between the grounding phases when actual stimulation takes place. This is shown in **Fig. 5** where the current monitoring element is implemented with a (precise) sensing resistor Rₛₑₙₛₑ in between high-voltage rail V_{HIGH} and switch S₇. When switch S₇ is closed, during the stimulation phases of the stimulation, the current Iₛₑₙₛₑ through the sensing resistor Rₛₑₙₛₑ equals the current I_{CCAN} absorbed from or delivered to the CAN by the CAN capacitor. The resulting sense voltage Vₛₑₙₛₑ = Rₛₑₙₛₑ I_{CCAN} can be amplified, digitized and analysed by the MCU for follow-up actions if needed.

For example, in the embodiment of Fig. 1A, for unipolar stimulation, the current of each phase of each stimulation pulse flows through the sensing resistor and this current can be analysed by the MCU. If the current level of one or more phases is out of the specified range, the MCU can try to adjust the involved pulse phase current and if that fails, the stimulation can be stopped and it can be flagged which current sources of which output stages of the stimulation engine are faulty.

The advantage of this second monitoring element is that only a single monitor is needed to monitor all (e.g. 16) output stages of the stimulation engine instead of incorporating a monitoring element in each individual output stage. In this way, a power-efficient and area and volume friendly monitoring function can be realized.

### References

- 10: control circuit
- 12: high voltage rail
- 14: (voltage) rail
- 16: H-bridge
- 18: branch
- 18a: node
- 20: branch
- 20a: node
- 22: branch
- 22a: node
- 24: branch
- 24a: node
- 24b: node
- 26: branch
- 28: branch

- ADC: analog-digital-converter
- CAN: can, housing, housing portion
- C_{CAN}: capacitor
- GND: ground
- I₁: first current source
- I₂: second current source
- I₃: current source
- I₄: third current source
- R₁: resistor
- R₂: resistor
- S₁: first switch
- S₂: second switch
- S₃: switch
- S₄: switch
- S₅: switch
- S₆: switch
- S₇: switch
- S₈: switch
- T: tissue

- 10': control circuit
- 12': high voltage rail
- 14': another rail
- 16': H-bridge
- 18a': node
- 20a': node
- 22': branch
- 22a': branch
- 24': branch
- 24a': node
- 24b': node
- 26': branch
- 28': branch

- 10": control circuit
- 12": high voltage rail
- 14": another rail
- 18a": node
- 24": branch
- 24a": node
- 24b": node
- 26": branch
- 28": branch

- ADC": analog-digital-converter
- CAN": can housing, housing portion
- C_{CAN}": capacitor
- GND": ground
- R₁": resistor
- R₂": resistor
- S₆": switch
- S₇": switch
- T": tissue

## Claims

1. A control circuit (10, 10', 10") for a neurostimulator, especially for an implantable pulse generator for a neurostimulator, comprising and/or being connected to at least one energy source, at least one housing (CAN, CAN', CAN") or housing portion (CAN, CAN', CAN") and/or at least one stimulation node and/or at least one stimulation surface configured to be in contact with tissue of a patient, at least one capacitor (C_{CAN}, C_{CAN'}, C_{CAN"}) being directly or indirectly connected with the housing (CAN, CAN', CAN") or housing portion (CAN, CAN', CAN") and/or the stimulation node and/or the stimulation surface, the control circuit (10, 10', 10") and/or the capacitor (C_{CAN}, C_{CAN'}, C_{CAN"}) being arranged and configured such that the housing (CAN, CAN', CAN") or housing portion (CAN, CAN', CAN") and/or the stimulation node and/or the stimulation surface is kept at a predefined voltage or kept within a predefined voltage range by means of the control circuit (10, 10', 10") and/or the capacitor (C_{CAN}, C_{CAN'}, C_{CAN"}),
wherein the control circuit (10, 10', 10") comprises at least one energy controlling element, which is configured such that it uses energy from or returns energy to the energy source to keep the housing (CAN, CAN', CAN") and/or the housing portion (CAN, CAN', CAN") and/or the stimulation node and/or the stimulation surface at a predefined voltage or within a predefined voltage range.

2. The control circuit (10, 10', 10") according to claim 1, **characterized in that** the control circuit (10, 10', 10") further comprises a monitoring element.

3. The control circuit (10, 10', 10") according to claim 2, **characterized in that** the monitoring element is configured such that by means of the monitoring element the voltage across the capacitor (C_{CAN}, C_{CAN'}, C_{CAN"}) and/or the voltage of the housing (CAN, CAN', CAN") and/or housing portion (CAN, CAN', CAN") and/or the voltage of the stimulation node and/or the stimulation surface is monitored and/or the current through the capacitor (C_{CAN}, C_{CAN'}, C_{CAN"}) and/or the housing (CAN, CAN', CAN") and/or the housing portion (CAN, CAN', CAN") and/or the stimulation node and/or the stimulation surface is monitored and/or the voltage and/or the current and/or the power of the energy source is monitored.

4. The control circuit (10, 10', 10") according to one of the preceding claims, **characterized in that** the control circuit (10) comprises at least one of the following elements: a current source, a switch, a voltage source, a ground (GND), and a bridge circuit.

5. The control circuit (10, 10', 10") according to claim 4, **characterized in that** the housing (CAN, CAN', CAN") and/or housing portion (CAN, CAN', CAN") and/or the stimulation node and/or the stimulation surface is part of the bridge circuit.

6. The control circuit (10, 10', 10") according to claim 4 or claim 5, **characterized in that** the bridge circuit comprises or is an H-bridge (16, 16', 16") circuit.

7. The control circuit (10, 10', 10") according to one of the preceding claims, **characterized in that** a supply voltage within a range from about 0 V to approx. 50 V, especially from approx. 5 V to approx. 30 V, preferably from approx. 20 V to approx. 30 V, is supplied by a voltage supply (V_{HIGH}).

8. The control circuit (10, 10', 10") according to claim 1, **characterized in that** the control circuit (10, 10', 10") comprises the at least a capacitor (C_{CAN}, C_{CAN'}, C_{CAN"}), a monitoring element, a switch (S₇) being arranged in a branch (24) connecting the capacitor (C_{CAN}, C_{CAN'}, C_{CAN"}) with a voltage supply (V_{HIGH}) and/or another voltage source, and a further switch (S₆) being arranged in a branch connecting the capacitor (C_{CAN}, C_{CAN'}, C_{CAN"}) with a rail (14).

9. The control circuit (10, 10', 10") according to claim 8, **characterized in that** there is a first current source and at least one further current source (I₁, I₂, I₃, I₄).

10. The control circuit (10, 10', 10") according to claim 9, **characterized in that** the control circuit (10, 10', 10") further comprises a first control switch (S₃), and a second control switch (S₄) connected in series, wherein the first control switch (S₃) and the second control switch (S₄) are arranged between a first current source (I₃) and a second current source (I₄).

11. The control circuit (10, 10', 10") according to one of the preceding claims, **characterized in that** the control circuit (10, 10', 10") further comprises a further switch (S₆) for connecting the capacitor (C_{CAN}) and/or the housing (CAN, CAN', CAN") or housing portion (CAN, CAN', CAN") to ground (GND) and/or another rail (14).

12. The control circuit (10, 10', 10") according to one of the preceding claims, **characterized in that** the control circuit (10, 10', 10") is directly or indirectly connected to a stimulation generation module of an implantable pulse generator, wherein the stimulation generation module is powered from a single energy source.

13. The control circuit (10, 10', 10") according to claim 12, **characterized in that** due to the voltage being controllable and/or settable at the housing (CAN, CAN', CAN") or housing portion (CAN, CAN', CAN") and/or the stimulation node and/or the stimulation surface configured to be in contact with tissue of a patient, at least one stimulation current source or output stage of the stimulation generation module can remain in its high-ohmic output range and/or its compliance region during stimulation.

## Patentansprüche

1. Steuerungsschaltung (10, 10', 10") für einen Neurostimulator, insbesondere für einen implantierbaren Impulsgenerator für einen Neurostimulator, die mindestens eine Energiequelle, mindestens ein Gehäuse (CAN, CAN', CAN") oder einen Gehäuseabschnitt (CAN, CAN', CAN") und/oder mindestens einen Stimulationsknoten und/oder mindestens eine Stimulationsfläche, die dafür ausgelegt ist, in Kontakt mit Gewebe eines Patienten zu sein, aufweist oder damit verbunden ist, wobei mindestens ein Kondensator (C_{CAN}, C_{CAN'}, C_{CAN"}) direkt oder indirekt mit dem Gehäuse (CAN, CAN', CAN") oder dem Gehäuseabschnitt (CAN, CAN', CAN") und/oder dem Stimulationsknoten und/oder der Stimulationsfläche verbunden ist, wobei die Steuerungsschaltung (10, 10', 10") und/oder der Kondensator (C_{CAN}, C_{CAN'}, C_{CAN"}) so eingerichtet und ausgelegt sind, dass das Gehäuse (CAN, CAN', CAN") oder der Gehäuseabschnitt (CAN, CAN', CAN") und/oder der Stimulationsknoten und/oder die Stimulationsfläche mithilfe der Steuerungsschaltung (10, 10', 10") und/oder des Kondensators (C_{CAN}, C_{CAN'}, C_{CAN"}) auf einer vordefinierten Spannung oder innerhalb eines vordefinierten Spannungsbereichs gehalten sind,
wobei die Steuerungsschaltung (10, 10', 10") mindestens ein Energiesteuerungselement aufweist, das so ausgelegt ist, dass es Energie von der Energiequelle verwendet oder Energie an die Energiequelle zurückschickt, um das Gehäuse (CAN, CAN', CAN") und/oder den Gehäuseabschnitt (CAN, CAN', CAN") und/oder den Stimulationsknoten und/oder die Stimulationsfläche auf einer vordefinierten Spannung oder innerhalb eines vordefinierten Spannungsbereichs zu halten.

2. Steuerungsschaltung (10, 10', 10") nach Anspruch 1, **dadurch gekennzeichnet, dass** die Steuerungsschaltung (10, 10', 10") darüber hinaus ein Überwachungselement aufweist.

3. Steuerungsschaltung (10, 10', 10") nach Anspruch 2, **dadurch gekennzeichnet, dass** das Überwachungselement so ausgelegt ist, dass mittels des Überwachungselements die Spannung am Kondensator (C_{CAN}, C_{CAN'}, C_{CAN"}) und/oder die Spannung des Gehäuses (CAN, CAN', CAN") und/oder des Gehäuseabschnitts (CAN, CAN', CAN") und/oder die Spannung des Stimulationsknotens und/oder der Stimulationsfläche überwacht wird und/oder der Strom durch den Kondensator (C_{CAN}, C_{CAN'}, C_{CAN"}) und/oder das Gehäuse (CAN, CAN', CAN") und/oder den Gehäuseabschnitt (CAN, CAN', CAN") und/oder den Stimulationsknoten und/oder die Stimulationsfläche überwacht wird und/oder die Spannung und/oder der Strom und/oder das Leistungsvermögen der Energiequelle überwacht wird.

4. Steuerungsschaltung (10, 10', 10") nach einem der vorhergehenden Ansprüche, **dadurch gekennzeichnet, dass** die Steuerungsschaltung (10) mindestens eines der folgenden Elemente aufweist: eine Stromquelle, einen Schalter, eine Spannungsquelle, eine Masse (GND) und eine Brückenschaltung.

5. Steuerungsschaltung (10, 10', 10") nach Anspruch 4, **dadurch gekennzeichnet, dass** das Gehäuse (CAN, CAN', CAN") und/oder der Gehäuseabschnitt (CAN, CAN', CAN") und/oder der Stimulationsknoten und/oder die Stimulationsfläche Teil der Brückenschaltung sind.

6. Steuerungsschaltung (10, 10', 10") nach Anspruch 4 oder Anspruch 5, **dadurch gekennzeichnet, dass** die Brückenschaltung eine H-Brückenschaltung (16, 16', 16") aufweist oder ist.

7. Steuerungsschaltung (10, 10', 10") nach einem der vorhergehenden Ansprüche, **dadurch gekennzeichnet, dass** von einer Spannungsversorgung (V_{HIGH}) eine Versorgungsspannung in einem Bereich von ca. 0 V bis ca. 50 V, insbesondere von ca. 5 V bis ca. 30 V, vorzugsweise von ca. 20 V bis ca. 30 V geliefert wird.

8. Steuerungsschaltung (10, 10', 10") nach Anspruch 1, **dadurch gekennzeichnet, dass** die Steuerungsschaltung (10, 10', 10") den mindestens einen Kondensator (C_{CAN}, C_{CAN'}, C_{CAN"}), ein Überwachungselement, einen Schalter (S₇), der in einem Zweig (24) angeordnet ist, welcher den Kondensator (C_{CAN}, C_{CAN'}, C_{CAN"}) mit einer Spannungsversorgung (V_{HIGH}) und/oder einer anderen Spannungsquelle verbindet, und einen weiteren Schalter (S₆) aufweist, der in einem Zweig angeordnet ist, welcher den Kondensator (C_{CAN}, C_{CAN'}, C_{CAN"}) mit einer Schiene (14) verbindet.

9. Steuerungsschaltung (10, 10', 10") nach Anspruch 8, **dadurch gekennzeichnet, dass** eine erste Stromquelle und mindestens eine weitere Stromquelle (I₁, I₂, I₃, I₄) vorhanden sind.

10. Steuerungsschaltung (10, 10', 10") nach Anspruch 9, **dadurch gekennzeichnet, dass** die Steuerungsschaltung (10, 10', 10") darüber hinaus einen ersten Steuerschalter (S₃) und einen zweiten Steuerschalter (S₄) aufweist, die in Reihe geschaltet sind, wobei der erste Steuerschalter (S₃) und zweite Steuerschalter (S₄) zwischen einer ersten Stromquelle (I₃) und einer zweiten Stromquelle (I₄) angeordnet sind.

11. Steuerungsschaltung (10, 10', 10") nach einem der vorhergehenden Ansprüche, **dadurch gekennzeichnet, dass** die Steuerungsschaltung (10, 10', 10") darüber hinaus einen weiteren Schalter (S₃) aufweist, um den Kondensator (C_{CAN}) und/oder das Gehäuse (CAN, CAN', CAN") oder den Gehäuseabschnitt (CAN, CAN', CAN") mit Masse (GND) und/oder einer weiteren Schiene (14) zu verbinden.

12. Steuerungsschaltung (10, 10', 10") nach einem der vorhergehenden Ansprüche, **dadurch gekennzeichnet, dass** die Steuerungsschaltung (10, 10', 10") direkt oder indirekt mit einem Stimulationsgenerierungsmodul eines implantierbaren Impulsgenerators verbunden ist, wobei das Stimulationsgenerierungsmodul aus einer einzigen Energiequelle mit Strom versorgt wird.

13. Steuerungsschaltung (10, 10', 10") nach Anspruch 12, **dadurch gekennzeichnet, dass** aufgrund dessen, dass die Spannung am Gehäuse (CAN, CAN', CAN") oder Gehäuseabschnitt (CAN, CAN', CAN") und/oder am Stimulationsknoten und/oder an der Stimulationsfläche, die dafür ausgelegt ist, in Kontakt mit Gewebe eines Patienten zu sein, steuerbar und/oder einstellbar ist, mindestens eine Stimulationsstromquelle oder Ausgangsstufe des Stimulationsgenerierungsmoduls während der Stimulation in ihrem hochohmigen Ausgangsbereich und/oder ihrem Compliance-Bereich bleiben kann.

## Revendications

1. Circuit de commande (10, 10', 10") pour un neurostimulateur, particulièrement pour un générateur d'impulsions implantable conçu pour un neurostimulateur, comprenant et/ou étant relié à au moins une source d'énergie, au moins un carter (CAN, CAN', CAN") ou une partie de carter (CAN, CAN', CAN") et/ou au moins un noeud de stimulation et/ou au moins une surface de stimulation configurée pour être en contact avec le tissu d'un patient, au moins un condensateur (C_{CAN}, C_{CAN'} , C_{CAN"}) étant directement ou indirectement relié au carter (CAN, CAN', CAN") ou à la partie de carter (CAN, CAN', CAN") et/ou au noeud de stimulation et/ou à la surface de stimulation, le circuit de commande (10, 10', 10") et/ou le condensateur (C_{CAN}, C_{CAN'}, C_{CAN"}) étant agencés et configuré de telle sorte que le carter (CAN, CAN', CAN") ou la partie de carter (CAN, CAN', CAN") et/ou le noeud de stimulation et/ou la surface de stimulation soient maintenus à une tension prédéfinie ou maintenus à l'intérieur d'une plage de tensions prédéfinies au moyen du circuit de commande (10, 10', 10") et/ou du condensateur (C_{CAN}, C_{CAN'}, C_{CAN"}), dans lequel le circuit de commande (10, 10', 10") comprend au moins un élément de commande d'énergie qui est configuré de façon à utiliser l'énergie provenant de la source d'énergie ou à faire revenir l'énergie à cet endroit de façon à maintenir le carter (CAN, CAN', CAN") et/ou la partie de carter (CAN, CAN', CAN") et/ou le noeud de stimulation et/ou la surface de stimulation à une tension prédéfinie ou à l'intérieur d'une plage de tensions prédéfinies.

2. Circuit de commande (10, 10', 10") selon la revendication 1, **caractérisé en ce que** le circuit de commande (10, 10', 10") comprend en outre un élément de surveillance.

3. Circuit de commande (10, 10', 10") selon la revendication 2, **caractérisé en ce que** l'élément de surveillance est configuré de telle sorte que la tension traversant le condensateur (C_{CAN}, C_{CAN'} , C_{CAN"}) et/ou la tension du carter (CAN, CAN', CAN") et/ou de la partie de carter (CAN, CAN', CAN") et/ou la tension du noeud de stimulation et/ou de la surface de stimulation est surveillée au moyen de l'élément de surveillance et/ou que le courant passant à travers le condensateur (C_{CAN}, C_{CAN'}, C_{CAN"}) et/ou le carter (CAN, CAN', CAN") et/ou la partie de carter (CAN, CAN', CAN") et/ou le noeud de stimulation et/ou la surface de stimulation est surveillé et/ou que la tension et/ou le courant et/ou la puissance de la source d'énergie est surveillé.

4. Circuit de commande (10, 10', 10") selon l'une quelconque des revendications précédentes, **caractérisé en ce que** le circuit de commande (10) comprend au moins un des éléments suivants : une source de courant, un interrupteur, une source de tension, une terre (GND) et un circuit en pont.

5. Circuit de commande (10, 10', 10") selon la revendication 4, **caractérisé en ce que** le carter (CAN, CAN', CAN") et/ou la partie de carter (CAN, CAN', CAN") et/ou le noeud de stimulation et/ou la surface de stimulation fait partie du circuit en pont.

6. Circuit de commande (10, 10', 10") selon la revendication 4 ou la revendication 5, **caractérisé en ce que** le circuit en pont comprend ou est un circuit en pont en H (16, 16', 16").

7. Circuit de commande (10, 10', 10") selon l'une quelconque des revendications précédentes, **caractérisé en ce qu'**une tension d'alimentation située à l'intérieur d'une plage d'environ 0 V à environ 50 V, particulièrement d'environ 5 V à environ 30 V, de préférence d'environ 20 V à environ 30 V, est alimentée par une alimentation en tension (V_{HIGH}) .

8. Circuit de commande (10, 10', 10") selon la revendication 1, **caractérisé en ce que** le circuit de commande (10, 10', 10") comprend l'au moins un condensateur (C_{CAN}, C_{CAN'} , C_{CAN"}), un élément de surveillance, un interrupteur (S7) étant agencé dans une branche (24) reliant le condensateur (C_{CAN}, C_{CAN'}, C_{CAN"}) à une alimentation en tension (V_{HIGH}) et/ou à une autre source de tension et un autre interrupteur (Se) étant agencé dans une branche reliant le condensateur (C_{CAN}, C_{CAN'}, C_{CAN"}) à un rail (14) .

9. Circuit de commande (10, 10', 10") selon la revendication 8, **caractérisé en ce qu'**il comporte une première source de courant et au moins une autre source de courant (I₁, I₂, I₃, I₄).

10. Circuit de commande (10, 10', 10") selon la revendication 9, **caractérisé en ce que** le circuit de commande (10, 10', 10") comprend en outre un premier interrupteur de commande (S₃), et un second interrupteur de commande (S₄) reliés en série, dans lequel le premier interrupteur de commande (S₃) et le second interrupteur de commande (S₄) sont agencés entre une première source de courant (I₃) et une seconde source de courant (I₄).

11. Circuit de commande (10, 10', 10") selon l'une quelconque des revendications précédentes, **caractérisé en ce que** le circuit de commande (10, 10', 10") comprend en outre un autre interrupteur (S₆) permettant de relier le condensateur (C_{CAN}) et/ou le carter (CAN, CAN', CAN") ou la partie de carter (CAN, CAN', CAN") à la terre (GND) et/ou à un autre rail (14).

12. Circuit de commande (10, 10', 10") selon l'une quelconque des revendications précédentes, **caractérisé en ce que** le circuit de commande (10, 10', 10") est directement ou indirectement relié à un module de génération de stimulation d'un générateur d'impulsions implantable, dans lequel le module de génération de stimulation est alimenté en courant à partir d'une seule source d'énergie.

13. Circuit de commande (10, 10', 10") selon la revendication 12, **caractérisé en ce qu'**en raison de la tension commandable et/ou réglable au niveau du carter (CAN, CAN', CAN") ou de la partie de carter (CAN, CAN', CAN") et/ou du noeud de stimulation et/ou de la surface de stimulation configurée pour être en contact avec le tissu d'un patient, au moins une source de courant de stimulation ou un étage de sortie du module de génération de stimulation peut rester dans sa plage de sortie à haute résistance et/ou dans sa zone de compatibilité pendant la stimulation.
